# EUROPEAN PATENT SPECIFICATION

(11) **EP 1 939 626 B1**
(45) Date of publication and mention of the grant of the patent: **30.12.2009**
(21) Application number: 06797687.8
(22) Date of filing: 08.09.2006
(51) Int. Cl.: G01N 33/536, G01N 33/58, G01N 33/68, G01N 21/78

(54) **METHOD OF QUICKLY DETECTING ANTIGEN USING FLUORESCENCE CORRELATION SPECTROSCOPY OR FLUORESCENCE CROSS-CORRELATION SPECTROSCOPY**
VERFAHREN ZUM SCHNELLNACHWEIS VON ANTIGEN MITTELS FLUORESZENZKORRELATIONSSPEKTROSKOPIE ODER FLUORESZENZKREUZKORRELATIONSSPEKTROSKOPIE
PROCÉDÉ DE DÉTECTION RAPIDE D'UN ANTIGÈNE EN UTILISANT LA SPECTROSCOPIE À CORRELATION EN FLUORESCENCE OU LA SPECTROSCOPIE À CORRELATION CROISÉE EN FLUORESCENCE

(30) Priority: 12.09.2005 JP 2005264394
(43) Date of publication of application: 02.07.2008
(73) Proprietor: Japan Science and Technology Agency, Kawaguchi-shi, Saitama 332-0012 (JP); National University Corporation Hokkaido University, Sapporo-shi Hokkaido 060-0808 (JP)
(72) Inventor: SAITO, Kenta National Univ. Corp. Hokkaido University,, Sapporo-shi, Hokkaido 060-0812 (JP); SAKATA, Hiroshi 5-10-602, Kita 14-jyo 1-chome,, Hokkaido 065-0014 (JP); FUJII, Fumihiko, Osaka 560-0054 (JP); KINJO, Masataka c/o National Univ. Corp. Hokkaido Univ., Sapporo-shi, Hokkaido 060-0810 (JP); TAMURA, Mamoru c/o Nat. Univ. Corp. Hokkaido Univ.,, N.11-ch., Kita-Ku, Sapporo-shi, Hokkaido 001-0021 (JP)
(74) Representative: Richards, William John
(86) International application number: PCT/JP2006/317832
(87) International publication number: WO 2007/032266

(56) References cited:
- EP-A- 1 767 940
- JP-A- 2001 194 305
- JP-A- 2005 172 460
- GIESE A ET AL: "PUTTING PRIONS INTO FOCUS: APPLICATION OF SINGLE MOLECULE DETECTION TO THE DIAGNOSIS OF PRION DISEASES" ARCHIVES OF VIROLOGY, NEW YORK, NY, US, no. 16, SUPPL, 1 January 2000 (2000-01-01), pages 161-171, XP001000222 ISSN: 0304-8608
- FUJII ET AL: "Detection of prion protein immune complex for bovine spongiform encephalopathy diagnosis using fluorescence correlation spectroscopy and fluorescence cross-correlation spectroscopy" ANALYTICAL BIOCHEMISTRY, ACADEMIC PRESS INC. NEW YORK, vol. 370, no. 2, 1 October 2007 (2007-10-01), pages 131-141, XP022278419 ISSN: 0003-2697
- SAKATA K.: 'Shinkei Hensei Shikkan Kenkyu no Saizensen Keiko Sokan Bunkoho (FCS) o Mochiita Kogen Kotai Hanno Kaiseki Oyobi Kentai Kenshutsu' BIO INDUSTRY vol. 21, no. 4, 2004, pages 52 - 59, XP003010523
- KOGUN E.: 'BSE no Zen Jido Kensa Sochi Ikita Ushi kara Prions o Toraeru' JST NEWS vol. 1, no. 5, February 2005, pages 10 - 11, XP003010524
- FOELDES-PAPP Z.: 'Ultrasensitive detection and identification of fluorescent molecules by FCS: Impact for immunobiology' PNAS vol. 98, no. 20, 2001, pages 11509 - 11514, XP002237790
- GIESE A.: 'Putting prions into focus: application of single molecule detection to the diagnosis of prion diseases' ARCHIVES OF VIROLOGY no. SUPPLEMENT 16, 2000, pages 161 - 171, XP001000222

## Description

### Technical Field

The present invention relates to a method of quickly detecting an antigen using fluorescence correlation spectroscopy (FCS) or fluorescence cross-correlation spectroscopy (FCCS). The present invention particularly relates to a method of quickly detecting an antigen at an arbitrary concentration in a sample using fluorescence correlation spectroscopy or fluorescence cross-correlation spectroscopy, without a multi-stage examination of the concentration ratio between a detection reagent and the antigen to be detected.

### Background Art

In the use of natural product-derived food materials or feed materials, the presence of a harmful protein, a pathogenic protein, or the like contained in those materials has raised a concern in recent years. Examples of harmful proteins include a controversial allergen protein contained in food materials such as buckwheat, wheat, and rice. Examples of pathogenic proteins include a pathogenic protein such as a controversial abnormal prion (infectious) contained in materials for edible meat and meat-and-bone meal. To explain it by illustration, an abnormal prion taken as a representative example of a pathogenic protein of concern in recent years is a protein that causes prion disease typified by bovine spongiform encephalopathy (BSE). A normal prion protein commonly present in animal brain and neural cell membrane surface is a glycoprotein with a molecular weight of approximately thirty-three thousands to thirty-five thousands (33 to 35 kDa), and its infectious prion protein form is intracellularly accumulated in the brain (Lait, 76: 571-578, 1996). Abnormal prions, after entering into an animal body, convert normal prions produced at particular sites in the body into abnormal prions, resulting in the accumulation of the abnormal prions at those particular sites. The accumulation of the abnormal prions in the brain renders the brain spongiform, leading to animal death.

The use of such food materials or feed materials requires detecting and assaying a harmful protein (e.g., an allergen protein) or pathogenic protein contained in food materials or feed materials and avoiding the use of those containing harmful proteins or pathogenic proteins, for preventing humans or animals from ingesting a harmful protein (e.g., an allergen protein) or pathogenic protein contained in those materials.

Conventionally, an immunoassay such as ELISA (enzyme-linked immunosorbent assay) or western blotting (immunoblotting) has been used in the assay of natural biological proteins such as a prion (abnormal). However, to perform detection and assay of a prion by a conventional method, for example, ELISA or western blotting, the conventional method requires initially performing, for example, a procedure of digesting and removing in advance a normal prion from a test sample by proteinase K treatment, for detecting an abnormal prion separately from a normal prion. Further, western blotting requires performing electrophoresis. Thus, this method involves complexities and takes much time. Therefore, it presents a problem of being unsuitable for practicing a test on a large number of samples in a short time. Furthermore, for achieving necessary sensitivity, ELISA requires, for example, subjecting a sample after proteinase K treatment to denaturation treatment with guanidine thiocyanate and performing primary denaturation treatment with SDS and a protein concentration procedure by methanol treatment before the deaggregation of the prion protein, and also requires performing centrifugation before the methanol treatment and before the treatment with guanidine thiocyanate, respectively. This centrifugation procedure takes much time. The method must perform such complicated treatment and therefore presents a problem of being unsuitable for practicing a test on a large number of samples in a short time.

Thus, to improve the problems of ELISA or western blotting used in the detection and assay of a prion, some methods have been proposed recently. For example, Japanese Laid-Open Patent Application No. 10-267928 has disclosed an immuno-PCR method to detect an abnormal prion protein with high sensitivity, wherein an anti-prion protein antibody is used and labeled with an arbitrary DNA fragment, which is detected by PCR. Further, Japanese Laid-Open Patent Application No. 2003-130880 has disclosed a method of immunoassaying an abnormal prion with high sensitivity without performing a time-consuming electrophoresis or centrifugation procedure of the conventional ELISA or western blotting method. In this method, a first antibody participating in an antigen/antibody reaction with an abnormal prion treated with a denaturing agent, or an antigen-binding fragment thereof is immobilized on magnetic particles and used as an immunoassay reagent for an abnormal prion.

These methods are modifications of the conventional ELISA or western blotting method and however, still must undergo a variety of treatments. Thus, these methods are not necessarily sufficient for conveniently and quickly detecting and assaying an antigenic protein such as a prion. Moreover, these detection and assay methods are less-than-suitable methods for automatically or semi-automatically performing treatment steps for detection and assay, and assaying large amounts of samples.

On the other hand, fluorescence correlation spectroscopy (FCS) has been known in recent years as an analysis method that is frequently used particularly in the analysis and the like of molecules derived from organisms and can detect and assay, in almost real time, the physical parameters of protein molecules such as number, sizes, or shapes without undergoing a step of physical separation of a sample (Chem. Phys., 4, 390-401, 1974; Biopolymers, 13, 1-27, 1974; Physical Rev. A, 10: 1938-1945, 1974; in Topics in Fluorescence Spectroscopy, 1, pp. 337-378, Plenum Press, New York and London, 1991; and R. Rigler, E. S. Elson (Eds.), Fluorescence Correlation Spectroscopy. Theory and Applications, Springer, Berlin, 2001). FCS is practiced by capturing, within an exceedingly small region, the Brownian motions of fluorescent-labeled target molecules in a medium by a laser confocal scanning microscope system and thereby analyzing the diffusion time from the fluctuation of fluorescence intensity and assaying the physical parameters of the target molecules (the number and sizes of the molecules). Analysis by such FCS, which capturesmolecularfluctuation within an exceedingly small region, serves as an effective means in specifically detecting intermolecular interaction with high sensitivity.

More specifically, with an FCS assay, the fluorescence intensity of an exceedingly small region (about 400 nm in diameter, about 2 µm in axial length, and up to 10⁻¹⁶L in volume) in a sample can be detected by using a confocal optics system. Since this region is an open system, molecules go into and out of the region, and a fluctuation is caused in fluorescence intensity according to the number of molecules. The fluctuation is averaged and becomes smaller as the number of molecule increases, and becomes faster as the molecular diffusion rate increases. By analyzing this fluctuation using the correlation function, information can be obtained regarding the number and sizes of the molecules. With an FCS assay, it is possible to analyze in real time the information regarding molecules without undergoing separation and purification and thus possible to screen a small amount of target molecules from vast amounts of samples. The feature of an FCS assay used in the detection and assay of a protein or the like contained in a biological sample is that the concentrations or intermolecular interactions of fluorescent-labeled target molecules contained in a solution can be monitored in almost real time without undergoing a physical separation step. Therefore, a detection system using FCS can avoid a complicated Bound/Free separation step required for conventional analysis means (e.g., ELISA) that have been predominantly used in biomolecule detection systems. Thus, this technique can assay large amounts of samples with high sensitivity in a short time and is also suitable for an automatic assay.

To detect an antigenic protein or the like by using FCS, a fluorescent-labeled antibody molecule is used, and antigen/antibody reaction between the fluorescent-labeled antibody and the antigenic protein is utilized. Analysis is performed by utilizing a difference in diffusion rate depending on the shapes and molecular weights of the fluorescent-labeled antibody and an antigen/antibody complex molecule formed by the antigen/antibody reaction of the fluorescent-labeled antibody and the antigenic protein. In this context, the diffusion rate (diffusion constant or D) refers to an area where molecules are freely diffused per unit time. On the other hand, the diffusion time (DT or τ_{D}) refers to time required for molecules to pass through a focal region determined depending on an apparatus.

Thus, the accurate assay of an antigenic protein or the like in a sample by FCS requires using a combination of an antigen and an antibody that causes a significant difference between the diffusion rate of the labeled antibody and the diffusion rate of an antigen/antibody complex formed by the antigen/antibody reaction of the labeled antibody and the antigenic protein. Thus, FCS could previously detect only extremely limited types of antigenic proteins or the like due to this requirement. Conventional means for solving this problem comprised applying a variety of modifications to an antigen/antibody complex in consideration of the shapes and molecular weights of the antigen and the antibody to provide a significant difference in diffusion rate (Japanese Laid-Open Patent Application No. 2001-272404 and Japanese Patent No. 3517241). However, even if these methods were used, there were limitations on an object to be detected to which the detection method by FCS was applicable.

Recently, as a method of detecting and assaying a substance using fluorescence spectroscopy, Fluorescence Cross-Correlation Spectroscopy (FCCS) is known as well as FCS (Biophysical Journal, 72: 1878-1886, 1997; Current Pharmaceutical Biotechnology, 5: 199-204, 2004). With an FCCS assay, two kinds of fluorescent intensities in an exceedingly small region in a sample can be detected using two kinds of lasers and two detectors. By analyzing the signals using the cross-correlation function, the correlation between the two kinds of signals can be seen. It has been proven that an FCCS assay is approximately 10 times more sensitive than an FCS assay, because an FCCS assay analyzes only signals that correlate with one another. Detecting an antigen using FCCS requires the use of (1) a fluorescent-labeled antibody and (2) a fluorescent-labeled antibody that recognizes a different epitope region, regardless of the molecular weight of the antigen.

Meanwhile, when using an FCS or FCCS assay to detect and assay a substance, usually, the concentration of the substance to be detected in a sample is often unknown. However, the problem is that, when using an FCS and FCCS assay to detect the binding of a fluorescent-labeled substance and the substance to be detected so as to assay the substance to be detected, a precise detection and assay of the substance to be detected is not possible unless an aptitude concentration ratio between the fluorescent-labeled substance and the substance to be detected is set. Therefore, when the concentration of the substance to be detected in a sample was unknown, it was necessary to perform a multi-stage dilution to examine the presence or absence of the substance to be detected (Japanese Laid-Open Patent Application No. 2005-43317; Japanese Laid-Open Patent Application No. 2005-98876). This resulted in a time-consuming assay, and thus, in such cases in the past, there was a problem that a quick detection and assay was difficult even with the use of FCS or FCCS assay. However, as far as the current situation is concerned, no effective measures have been developed heretofore.

Patent Document 1: Japanese Laid-Open Patent Application No. 10-267928
Patent Document 2: Japanese Laid-Open Patent Application No. 2001-272404
Patent Document 3: Japanese Laid-Open Patent Application No. 2003-130880
Patent Document 4: Japanese Laid-Open Patent Application No. 2005-43317
Patent Document 5: Japanese Laid-Open Patent Application No. 2005-98876
Patent Document 6: Japanese Patent No. 3517241
Non-Patent Document 1: Lait, 76: 571-578, 1996
Non-Patent Document 2: Chem. Phys., 4, 390-401, 1974
Non-Patent Document 3: Biopolymers, 13, 1-27, 1974
Non-Patent Document 4: Physical Rev. A, 10: 1938-1945, 1974
Non-Patent Document 5: in Topics in Fluorescence Spectroscopy, 1, pp. 337-378, Plenum Press, New York and London, 1991
Non-Patent Document 6: R. Rigler, E. S. Elson (Eds.), Fluorescence Correlation Spectroscopy, Theory and Applications, Springer, Berlin, 2001
Non-Patent Document 7: Biophysical Journal, 72: 1878-1886, 1997
Non-Patent Document 8: Current Pharmaceutical Biotechnology, 5: 199-204, 2004

### Disclosure of the Invention

### Object to be solved by the Invention

An object of the present invention is to provide a method of quickly detecting an antigen at an arbitrary concentration in an antigen sample using fluorescence correlation spectroscopy (FCS) or fluorescence cross-correlation spectroscopy (FCCS), without a multi-stage examination of the concentration ratio between a detection reagent and an antigen to be detected, particularly when the concentration of the antigen in the sample is unknown, in the method of detecting an antigen using FCS or FCSS.

### Means to solve the object

During diligent studies on a method of quickly detecting a pathogenic protein antigen in a biological protein sample or a harmful protein antigen contained in food material using FCS or FCSS by utilizing an antigen-antibody reaction, the present inventors found that, in the case where the antigen concentration in a sample is unknown, an antigen at an arbitrary concentration in an antigen sample can be quickly detected using FCS or FCCS without a multi-stage examination of the concentration ratio between a detection reagent and the antigen to be detected, by: preparing (1) a series to which only a detection reagent such as a fluorescent-labeled antigen is added and (2) a series to which an antigen and the detection reagent are added to achieve a maximum concentration of a trimer consisting of the antigen and the antibody; performing a fluorescence spectroscopic analysis; and detecting the presence or absence of the antigen in the detection sample by the presence or absence of a trimer detection signal from a detector in the cases of (1) and (2), and thus completed the present invention.

More specifically, the present invention is comprised of preparing (1) a series to which only a detection reagent is added and (2) a series to which an antigen and the detection reagent are added to achieve a maximum trimer concentration; performing a fluorescence spectroscopic analysis; and quickly detecting the presence or absence of the antigen in the detection sample by the presence or absence of a trimer detection signal from a detector in the cases of (1) and (2), in a method of detecting an antigen by FCS or FCCS using as a detection reagent a fluorescent-labeled intact antibody or fluorescent-labeled antibody fragment targeted to an epitope of an antigen to be detected, and a non-fluorescent-labeled intact antibody or fluorescent-labeled intact antibody or fluorescent-labeled antibody fragment targeted to another epitope of the antigen.

In the present invention, a series of the above (2), to which an antigen and a detection reagent are added to achieve a maximum trimer concentration, is prepared by: calculating in advance dissociation constants Kd of the fluorescent-labeled intact antibody or fluorescent-labeled antibody fragment, and the non-fluorescent-labeled intact antibody or fluorescent-labeled intact antibody or fluorescent-labeled antibody fragment against an antigen to be detected; setting the concentration ratio between the detection reagent and the antigen at which a maximum trimer concentration is achieved; and preparing based on the concentration ratio "a series to which the antigen and the detection reagent are added to achieve a maximum trimer concentration".

The method of quickly detecting an antigen of the present invention can be advantageously applied to "a method of quickly detecting and/or assaying antigen by FCS, "developed and applied for a patent earlier by the present inventors, i.e., a method of quickly detecting and/or assaying an antigen by fluorescence correlation spectroscopy (FCS) comprising quickly and precisely detecting and/or assaying an antigen in a sample with a simple operation using a fluorescent-labeled antibody fragment targeted to an epitope of the antigen, and a non-fluorescent-labeled intact antibody targeted to another epitope of the antigen (Japanese Patent Application No. 2004-166440). Further, the method of quickly detecting an antigen of the present invention can also be applied to the detection of an antigen by fluorescence cross-correlation spectroscopy (FCCS) using fluorescent-labeled antibodies or fluorescent-labeled antibody fragments, each targeted to a different epitope of the antigen.

The principle of the method of quickly detecting an antigen by FCS or FCCS of the present invention will be explained hereinbelow.

### (Assay by FCS)

With an FCS assay, a fluorescence intensity of an exceedingly small region (about 4 00 nm in diameter, about 2 µm in axial length, and up to 10⁻¹⁶L in volume) in a sample can be detected by using a confocal optics system. Since this region is an open system, molecules go into and out of the region, and a fluctuation is caused in fluorescence intensity according to the number of molecules. The fluctuation is averaged and becomes smaller as the number of molecule increases, and becomes faster as the molecular diffusion rate increases. By analyzing this fluctuation using the correlation function, information can be obtained regarding the number and sizes of the molecules. With an FCS assay, it is possible to analyze in real time the information regarding molecules without undergoing separation and purification and thus possible to screen a small amount of target molecules from vast amounts of samples.

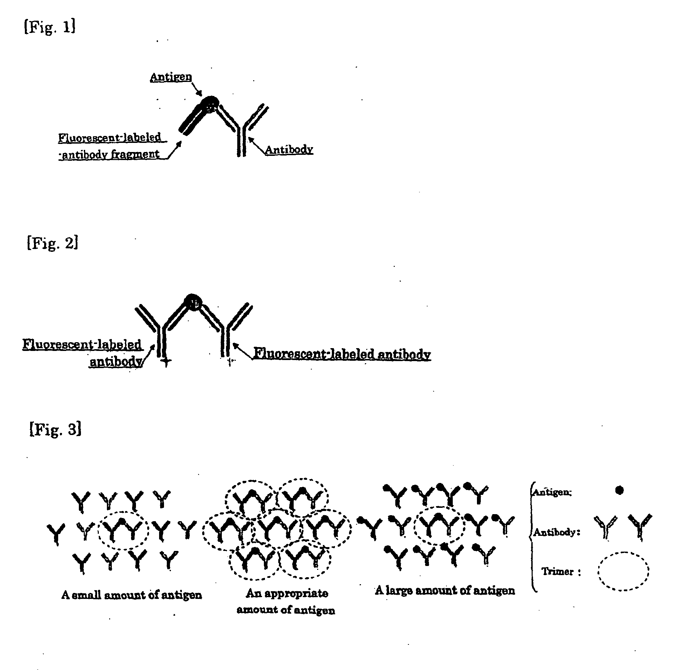

An antigen detection using FCS utilizes the fact that a fluorescent-labeled antibody captures an antigen thereby increases its molecular weight, resulting in delay in speed of the molecule passing the confocal region. When the object is an antigen of an arbitrary molecular weight, it is required to use (1) a fluorescent-labeled antibody fragment and (2) an antibody, that recognize different epitope regions of an antigen (Fig. 1). In this instance, as explained hereinbelow in the section of (Problem in a method of detecting an antigen using FCS and FCCS), the case where the antigen concentration is extremely high also results in a low trimer concentration as with the case of no antigen, causing a problem in detecting the antigen.

### (Assay by FCCS)

With an FCCS assay, two kinds of fluorescent intensities in an exceedingly small region in a sample can be detected by using two kinds of lasers and two detectors. By analyzing the signals using the cross-correlation function, the correlation between the two kinds of signals can be seen. It has been proven that an FCCS assay is approximately 10 times more sensitive than an FCS assay, because an FCCS assay analyzes only signals that correlate with one another. Detecting an antigen using FCCS requires the use of (1) a fluorescent-labeled antibody and (2) a fluorescent-labeled antibody that recognizes a different epitope region, regardless of the molecular weight of the antigen (Fig. 2). In this case, as explained hereinbelow in the section of (Problem in a method of detecting an antigen using FCS and FCCS) and similar to the case of an FCS assay, the case where antigen concentration is extremely high also results in a low trimer concentration as with the case of no antigen, causing a problem in detecting the antigen.

### (Problem in a method of detecting an antigen using FCS and FCCS)

When using FCS for detecting an antigen from a sample without setting a limitation on the molecular weight, (1) a fluorescent-labeled antibody fragment and (2) an antibody, that recognize different epitope regions of the antigen, are used. Further, when using FCCS for detecting an antigen, which is expected to be more sensitive than FCS, (1) a fluorescent-labeled antibody and (2) a fluorescent-labeled antibody that recognizes a different epitope region, are used. In both cases, an antigen in the sample is captured in a sandwiched-manner using the antibody and the antibody fragment, and the trimer thereof is to be detected (Figs. 1 and 2).

When these methods are used, the trimer concentration becomes low when the antigen concentration is low, while the trimer concentration also becomes low when the antigen concentration is extremely high (Fig. 3). Therefore, when the antigen concentration is unknown, the need arises to examine the presence or absence of the antigen by performing a multi-stage dilution for each sample to avoid a false-negative case. This results in a substantial loss of swiftness of the assay.

### (Principle of a method of quickly detecting an antigen)

In order to solve the above-mentioned problem in a method of detecting an antigen using FCS and FCCS, the following method is adopted in the present invention. In brief, if the dissociation constant Kd-value between an antigen and an antibody fragment or the like is known in advance, it will be possible to derive the concentration ratio between the detection reagent and the antigen at which a maximum trimer concentration is achieved. Based on this ratio, (1) a series to which only a detection reagent is added and (2) a series to which an antigen and the detection reagent are added to achieve a maximum trimer concentration, are prepared for each sample.

The detection results are determined as negative in the case of < (1) :without signal, (2) :with signal>, positive in the case of < (1) :with signal, (2) :with signal> or < (1) : with signal, (2):without signal>, and positive in the case of < (1) :without signal, (2) : without signal>. The use of the method of the present invention, with respect to an antigen at an arbitrary concentration in the sample, eliminates the need of adjusting the concentration of the antigen in a sample to be used for the test to an appropriate concentration or the need of preparing into multi-stage concentrations. This allows a quick detection of the antigen at an arbitrary concentration in the sample.

The method of quickly detecting an antigen of the present invention can be effectively used for such as the detection of a pathogenic protein antigen, particularly an abnormal prion, in a biological protein sample, or for the detection of a harmful protein antigen contained in food material.

More specifically, the present invention is comprised of a method of quickly detecting an antigen in a sample by fluorescence correlation spectroscopy or fluorescence cross-correlation spectroscopy, comprising a process of (1) adding to a test sample a fluorescent-labeled intact antibody or fluorescent-labeled antibody fragment targeted to an epitope of an antigen to be detected, and a non-fluorescent-labeled intact antibody or fluorescent-labeled intact antibody or fluorescent-labeled antibody fragment targeted to another epitope of the antigen as a detection reagent; letting an antigen-antibody reaction take place; and detecting a formed antigen-antibody trimer by fluorescence correlation spectroscopy or fluorescence cross-correlation spectroscopy and a process of (2) calculating a concentration ratio between the detection reagent and the antigen at which a maximum trimer concentration is achieved in advance and adding to the test sample the antigen and the detection reagent to achieve a maximum trimer concentration, in an amount calculated in advance from the concentration ratio between the detection reagent and the antigen at which a maximum trimer concentration is achieved; letting an antigen-antibody reaction take place; and detecting a formed antigen-antibody trimer by fluorescence correlation spectroscopy or fluorescence cross-correlation spectroscopy, which method further comprises combining a presence or absence of a trimer detection signal in the both processes to determine a presence or absence of the antigen in the test sample.

### Further embodiments:

The method of quickly detecting an antigen, wherein the test sample is a biological protein sample and the antigen to be detected is a pathogenic protein antigen.
The method of quickly detecting an antigen wherein the pathogenic protein antigen is an abnormal prion.
The method of quickly detecting an antigen, wherein the test sample is food material, and the antigen to be detected is a harmful protein antigen contained in the food material.
The method of quickly detecting an antigen, wherein the detection of an antigen by fluorescence correlation spectroscopy or fluorescence cross-correlation spectroscopy is performed without undergoing a process of physically separating the antigen contained in the test sample.

### Brief Description of Drawings

[Fig. 1]
   It is a schematic view showing the case where a trimer is formed with a fluorescent-labeled antibody fragment, a non-fluorescent-labeled intact antibody and an antigen, in the explanation of a method of quickly detecting an antigen using FCS of the present invention.
[Fig. 2]
   It is a schematic view showing the case where a trimer is formed with a fluorescent-labeled antibody, a fluorescent-labeled antibody and an antigen, in the explanation of a method of quickly detecting an antigen using FCCS of the present invention.
[Fig. 3]
   It is a schematic view illustrating the conditions of a trimer formation with antibodies and antigens, in the case of a low antigen concentration or in the case of an extremely high antigen concentration, in the explanation of a method of quickly detecting an antigen using FCS and FCCS of the present invention.
[Fig.4]
   It is a diagram showing the results of an Example of the present invention where (1) a series to which only a detection reagent was added and (2) a series to which the detection reagent and an appropriate amount of antigen were added, were prepared for the antigen of each concentration, and a trimer of antibodies and antigen was detected to determine whether a sample is antigen-positive or negative.

### Best Mode of Carrying Out the Invention

A method of quickly detecting an antigen in a sample by fluorescence correlation spectroscopy or fluorescence cross-correlation spectroscopy, comprising a process of (1) adding to a test sample a fluorescent-labeled intact antibody or fluorescent-labeled antibody fragment targeted to an epitope of an antigen to be detected, and a non-fluorescent-labeled intact antibody or fluorescent-labeled intact antibody or fluorescent-labeled antibody fragment targeted to another epitope of the antigen as a detection reagent; letting an antigen-antibody reaction take place; and detecting a formed antigen-antibody trimer by fluorescence correlation spectroscopy or fluorescence cross-correlation spectroscopy and a process of (2) calculating a concentration ratio between the detection reagent and the antigen at which a maximum trimer concentration is achieved in advance and adding to the test sample the antigen and the detection reagent to achieve a maximum trimer concentration, in an amount calculated in advance from the concentration ratio between the detection reagent and the antigen at which a maximum trimer concentration is achieved; letting an antigen-antibody reaction take place; and detecting a formed antigen-antibody trimer by fluorescence correlation spectroscopy or fluorescence cross-correlation spectroscopy, which method further comprises combining a presence or absence of a trimer detection signal in the both processes to determine a presence or absence of the antigen in the test sample.

In the present invention, an antigen and a detection reagent can be added to achieve a maximum trimer concentration by calculating in advance dissociation constants Kd of the fluorescent-labeled intact antibody or fluorescent-labeled antibody fragment, and the non-fluorescent-labeled intact antibody or fluorescent-labeled intact antibody or fluorescent-labeled antibody fragment against an antigen to be detected; setting the concentration ratio between the detection reagent and the antigen at which a maximum trimer concentration is achieved; and adding the antigen and the detection reagent in an amount set based on the concentration ratio.

More specifically, the present invention can be practiced by calculating in advance dissociation constants Kd of the fluorescent-labeled intact antibody or fluorescent-labeled antibody fragment, and the non-fluorescent-labeled intact antibody, or fluorescent-labeled intact antibody or fluorescent-labeled antibody fragment against an antigen to be detected; setting the concentration ratio between the detection reagent and the antigen at which a maximum trimer concentration is achieved; adding the antigen and the detection reagent in an amount set based on the concentration ratio thereby to prepare (1) a series to which only a detection reagent is added and (2) a series to which an antigen and the detection reagent are added to achieve a maximum trimer concentration; performing a fluorescence fluoroscopic analysis; and detecting the presence or absence of the antigen in the detection sample by the presence or absence of a trimer detection signal from a detector in the cases of (1) and (2).

### (Preparation of a series to which an antigen and a detection reagent are added to achieve a maximum trimer concentration)

The antigen concentration at which a maximum trimer concentration is achieved is calculated in advance from a titration curve using an antigen of a known concentration, for example, a recombinant bovine prion protein. The antigen of this concentration is added to prepare (2) a series to which a detection reagent and an antigen are added, while is not added to prepare (1) a series to which only the detection reagent is added, and then the presence or absence of the antigen of an unknown concentration in the sample is detected.

### (Preparation of an anti-protein antibody)

In the present invention, an antibody that specifically binds to an antigen is prepared in order to prepare a fluorescent-labeled antibody, fluorescent-labeled antibody fragment and non-fluorescent-labeled intact antibody used as a detection reagent. Examples of antibodies that specifically bind to an antigen used in the present invention include polyclonal antibodies, monoclonal antibodies and the like, among which, monoclonal antibodies are more preferred for their specificity. To prepare such antibodies against an antigen, firstly, the antigen to be detected is obtained by purification. The antigen can be prepared by isolation and purification from a donor source using a purification means known in the art. Alternatively, if the antigen is an antigenic protein having its amino acid sequence known in the art, the antigenic protein can be obtained by a genetic engineering approach whereby microorganisms, animal cells, or the like are used and allowed to produce the antigenic protein, followed by purification. The antigenic protein can be prepared, when possible, by a chemical peptide synthesis method. A synthesis means known in the art can be adopted for the chemical peptide synthesis. Examples thereof include azide, acid chloride, acid anhydride, mixed acid anhydride, DCC, active ester, carboimidazole, and oxidation-reduction methods.

To prepare antibodies against the antigen, animals or plants are sensitized to the antigen using a routine protocol. For example, to prepare monoclonal antibodies, any method that provides antibodies produced by cultures of continuous cell lines can be used, such as a hybridoma method (Nature 256, 495-497, 1975) , trioma method, human B cell hybridoma method (Immunology Today 4, 72, 1983), and EBV-hybridoma method (MONOCLONAL ANTIBODIES AND CANCER THERAPY, pp. 77-96, Alan R. Liss, Inc., 1985).

To prepare monoclonal antibodies against an antigen such as an antigenic protein, for example, mammals such as rat, mice, or rabbits are sensitized by administering the antigenic protein as an antigen to them. An adjuvant such as a Freund's complete adjuvant (FCA) or a Freund's incomplete adjuvant (FIA) can be used, if necessary. The immunization is performed mainly by an intravenous, hypodermic, or intraperitoneal injection. Moreover, a time interval between immunizations is not particularly limited, and 1 to 10 immunizations are performed at several-day to several-week intervals. One to sixty days after the final immunization day, antibody-producing cells are collected. Examples of the antibody-producing cells include spleen cells, lymph node cells, peripheral blood cells and the like. To obtain hybridomas, cell fusion is performed between the antibody-producing cells and myeloma cells. Generally available cell lines can be used as the myeloma cells to be fused with the antibody-producing cells. The cell lines used have drug selectivity and possess the property of being inviable in the unfused form in a HAT selective medium (which contains hypoxanthine, aminopterin, and thymidine) but viable therein only in the form fused with the antibody-producing cells.

Hybridomas of interest are selected from the cells after the cell fusion treatment. A usual cell culture method or ascites formation method can be adopted as a method of collecting monoclonal antibodies from the established hybridomas. When the method of collecting antibodies requires antibody purification, the antibodies can be purified by appropriately selecting a method known in the art such as ammonium sulfate precipitation, ion-exchange chromatography, gel filtration, and affinity chromatography or combining these methods. In the present invention, in addition to the antibodies thus prepared, commercially available already-prepared antibodies, if any, can be used as an antibody against the antigenic protein used in the present invention.

### (Preparation of a fluorescent-labeled antibody and fluorescent-labeled antibody fragment)

In the method of quickly detecting an antigen of the present invention, as a detection reagent for performing antigen/antibody reaction with an antigen and for detecting the antigen, a fluorescent-labeled antibody and fluorescent-labeled antibody fragment prepared from the antigen is used. In the present invention, as an antibody used in the preparation of a fluorescent-labeled antibody and fluorescent-labeled antibody fragment, an antibody that binds to a different antigenic epitope from that for a non-fluorescent-labeled intact antibody, fluorescent-labeled intact antibody, or fluorescent-labeled antibody fragment, which is also used in the present invention, is selected. The fluorescent-labeled antibody and fluorescent-labeled antibody fragment can be prepared by reducing an intact antibody or an intact antibody fragmented with an enzyme such as pepsin and papain, into a monomer with, for example, 2-mercaptomethylamine or 2-mercaptoethanol, followed by labeling. A fluorescent dye is used in the labeling. For example, a fluorescent dye such as fluorescein isothiocyanate (FITC) and Alexa 532 is used.

### (Detection and assay by FCS)

In the method of quickly detecting and/or assaying an antigen of the present invention, a fluorescent-labeled antibody fragment and non-fluorescent-labeled intact antibody as a detection reagent are added and mixed to a test sample. The test sample that has undergone antigen/antibody reaction is subjected to the detention and/or assay of the antigen by FCS. FCS is a method whereby the Brownian motions of fluorescent molecules in a solution are utilized to obtain the physical parameters of the molecules such as "sizes" or "number". The feature of FCS is that the concentrations or intermolecular interactions of fluorescent molecules contained in a solution can be monitored in almost real time without undergoing a physical separation step. Therefore, a detection system using FCS can avoid a complicated bound/free separation step required for conventional biomolecule detection systems (e.g., ELISA) performed predominantly in the past. Thus, large amounts of samples can be assayed automatically with high sensitivity in a short time. A variety of FCS techniques are known, and any method can be used in the present invention unless it hinders the detection and assay of an object to be detected and assayed by the present invention (Protein, Nucleic Acid and Enzyme, Vol. 44, No. 9, 1431-1438, 1999; Bio Industry, April issue, p. 52-59, 2003; Japanese Laid-Open Patent Application No. 2001-272404; and Japanese Patent No. 3517241).

### (Detection and assay by FCCS)

A variety of FCCS techniques are known, and any method can be used in the present invention unless it hinders the detection and assay of an object to be detected and assayed by the present invention (Biophysical Journal, 72, 1878-1886, 1997; Current Pharmaceutical Biotechnology, 5, 199-204, 2004; Biochem. Biophys. Res. Comm., 329, 1200-1207, 2005; Published Japanese translation of PCT international publication No. 2001-517800).

The present invention will be described in detail with reference to the following examples, while the scope of the present invention will not be limited to these exemplifications.

### Example 1

### (Materials and methods)

MF20 (Olympus) was used as an FCS and FCCS assay device, and a 384-well plate (Olympus) treated by blocking with N101 (Nippon Oil & Fats Co. Ltd) was used as an assay plate. Alexa 488-anti-prion antibody (1.0E-10M) and Alexa 633-anti-prion antibody (1.0E-10M) were used as a detection reagent, and a recombinant bovine-prion protein was used as an antigen. For the antigen of each concentration the followings were prepared: (1) a series to which only a detection reagent was added and (2) a series to which the detection reagent and an appropriate amount of the antigen were added.

### (Preparation of a series to which a detection reagent and an appropriate amount of antigen are added)

The antigen concentration at which a maximum trimer concentration is achieved was calculated to be about 4.8 nM from (1) the series to which only a detection reagent was added. The antigen and the detection reagent were added to a sample to attain this concentration, to be prepared as (2) a series to which the detection reagent and the antigen are added.

### (Detection of antigen)

For the antigen of each concentration, the followings were prepared: (1) a series to which only a detection reagent is added; and (2) a series to which the detection reagent and an appropriate amount of antigen are added. The detection reagent and the antigen were put into a plate so as to attain a given concentration, and then stirred with a pipette. After the plate was left at 37°C for 1 hour, measurements were carried out three times in 60 seconds at 488 nm at the laser power of 100 µW and at 633 nm at the laser power of 100 µW. An analysis was performed using MF20-associated software to derive various parameters such as the number of molecules.

### (Results)

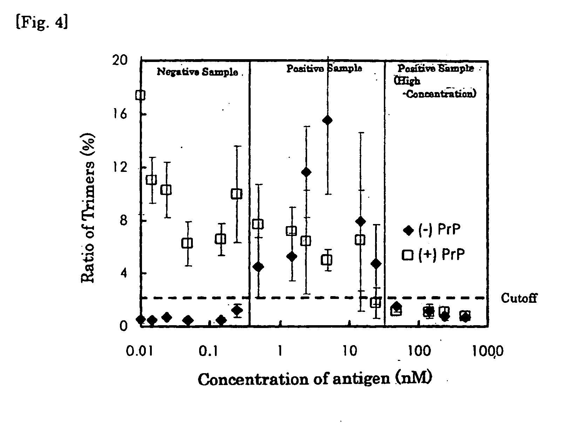

The results are shown in Fig. 4. In Fig. 4, the horizontal axis of the graph represents the antigen concentration and the vertical axis the rate of trimer to the total fluorescent molecules. Since the detection-limit value of FCCS is presently about 5x10⁻¹⁰M, the concentration of prion protein equal to or smaller than 5x10⁻¹⁰M is assumed to be negative. In addition, equal to or smaller than about 2% is considered to be no signal in the present data, based on the signal intensity of a negative control. When a given amount of antigen is not newly added [(-) PrP in the Figure] and when the antigen concentration is 1 to 10 nM, the molecular number of antigen is equal to those of two kinds of labeled antibodies, thus the rate of trimer is increased (Fig. 4, black dots). However, the rate of trimer decreases when the antigen concentration is low, and also when the antigen concentration is high (Fig. 4, black dots). Therefore, the case of a high antigen concentration is also determined to be negative.

Meanwhile, when the antigen is added in advance at a concentration that achieves a maximum trimer rate [(+) PrP in the Figure], trimer concentration increases if the antigen concentration is low and decreases if the antigen concentration is high (Fig. 4, gray dots). In this instance, the case of a high antigen concentration can be distinguished from a true-negative case. It is therefore possible to discriminate between negative and positive by combining for each sample (1) a series to which only a detection reagent is added and (2) a series to which an antigen and the detection reagent are added to achieve a maximum trimer concentration. In brief, the case of < (1) : without signal; (2) : with signal> can be determined as negative, the case of <(1) :with signal; (2) :with signal> or <(1) :with signal; (2) :without signal> as positive, and the case of <(1) :without signal; (2):without signal> as positive.

### Industrial Applicability

Recently, it is required at the social level that pathogenic proteins such as abnormal prion proteins and harmful proteins be detected. Presently, western blotting and ELISA are employed as methods of detecting abnormal prion proteins treated with a denaturant. However, western blotting requires performing cumbersome and time-consuming electrophoresis, and ELISA requires signal amplification and washing operations. In this context, antigen detections by FCS and FCCS comprise fewer steps as compared to the antigen detection methods employed in related fields and are therefore quicker and suitable for automation. Further, FCS and FCCS are economical due to a small amount of detection reagent because the sample volume required for the assay is several µl to several tens of µl. Therefore, it is highly effective to employ antigen detection methods by FCS and FCCS for detecting a pathogenic protein antigen and a harmful protein.

However, when antigen detection methods by FCS and FCCS were used for detecting an antigen from a sample of an unknown concentration, in order to avoid a false-negative case, it was necessary to set the antigen in the sample used for the test to an appropriate concentration, or to adjust each sample into multi-stage concentrations by performing a multi-stage dilution. This resulted in a substantial loss of swiftness of the assay. In this context, the detection method of the present invention solved this problem and enabled a quick detection method by FCS and FCCS without performing a multi-stage dilution of a sample of an unknown concentration. The detectionmethod of the present invention comprises a few steps, and is a quick and simple detection method which is applicable to a full automated system. It can be expected that the detection method of the present invention will be applied to a test for bovine spongiform encephalopathy (BSE) which is presently recognized as a social problem, and other tests of food, pharmaceuticals, cosmetics and the like based on an antigen-antibody reaction.

## Claims

1. A method of quickly detecting an antigen in a sample by fluorescence correlation spectroscopy or fluorescence cross-correlation spectroscopy, comprising a process of (1) adding to a test sample a fluorescent-labeled intact antibody or fluorescent-labeled antibody fragment targeted to an epitope of an antigen to be detected, and a non-fluorescent-labeled intact antibody or fluorescent-labeled intact antibody or fluorescent-labeled antibody fragment targeted to another epitope of the antigen as a detection reagent; letting an antigen-antibody reaction take place; and detecting a formed antigen-antibody trimer by fluorescence correlation spectroscopy or fluorescence cross-correlation spectroscopy and a process of (2) calculating a concentration ratio between the detection reagent and the antigen at which a maximum trimer concentration is achieved in advance and adding to the test sample the antigen and the detection reagent to achieve a maximum trimer concentration, in an amount calculated in advance from the concentration ratio between the detection reagent and the antigen at which a maximum trimer concentration is achieved; letting an antigen-antibody reaction take place; and detecting a formed antigen-antibody trimer by fluorescence correlation spectroscopy or fluorescence cross-correlation spectroscopy, which method further comprises combining a presence or absence of a trimer detection signal in the both processes to determine a presence or absence of the antigen in the test sample.

2. The method of quickly detecting an antigen according to claim 1, wherein the test sample is a biological protein sample and the antigen to be detected is a pathogenic protein antigen.

3. The method of quickly detecting an antigen according to claim 2, wherein the pathogenic protein antigen is an abnormal prion.

4. The method of quickly detecting an antigen according to claim 1, wherein the test sample is food material, and the antigen to be detected is a harmful protein antigen contained in the food material.

5. The method of quickly detecting an antigen according to any one of claims 1 to 4, wherein the detection of an antigen by fluorescence correlation spectroscopy or fluorescence cross-correlation spectroscopy is performed without undergoing a process of physically separating the antigen contained in the test sample.

## Patentansprüche

1. Verfahren zum schnellen Nachweis eines Antigens in einer Probe mit Hilfe von Fluoreszenz-Korrelations-Spektroskopie oder Fluoreszenz-Kreuzkorrelations-Spektroskopie,
bestehend aus einem Verfahrensschritt
(1), in dem zu einer Untersuchungsprobe ein auf ein Epitop des nachzuweisenden Antigens gerichteter fluoreszenzmarkierter intakter Antikörper oder ein fluoreszenzmarkiertes intaktes Antikörperfragment und ein auf ein anderes Epitop des Antigens gerichteter fluoreszenzmarkierter intakter Antikörper oder ein fluoreszensmarkiertes intaktes Antikörperfragment als Nachweisreagenz hinzugefügt wird;
Ablauf einer Antigen-Antikörper-Reaktion; und
Nachweis eines gebildeten Antigen-Antikörper-Trimers durch Fluoreszenz-Korrelations-Spektroskopie oder Fluoreszenz-Kreuzkorrelations-Spektroskopie, und einem Verfahrensschritt
(2), der Vorausberechnung eines Konzentrationverhältnisses zwischen dem Nachweisreagenz und dem Antigen, bei dem die maximale Trimerkonzentration erreicht wird, und Zusatz des Antigens und des Nachweisreagenz zur Untersuchungsprobe des Antigens und des Reagenz zur Untersuchungsprobe, um eine maximale Trimerkonzentration zu erreichen, in einer aus dem Konzentrationsverhältnis zwischen dem Nachweisreagenz und dem Antigen vorauszuberechnenden Menge, bei welcher eine maximale Trimerkonzentration erreicht wird;
Ablauf einer Antigen-Antikörper-Reaktion; und
Nachweis eines gebildeten Antigen-Antikörper-Trimers durch Fluoreszenz-Korrelations-Spektroskopie oder Fluoreszenz-Kreuzkorrelations-Spektroskopie,
**dadurch gekennzeichnet, dass**
das Verfahren weiterhin besteht aus der Kombination einer Präsenz oder Nicht-Präsenz eines Trimer-Nachweissignals bei beiden Verfahrensschritten, um eine Präsenz oder Nicht-Präsenz des Antigens in der Untersuchungsprobe festzustellen.

2. Verfahren zum schnellen Nachweis eines Antigens gemäß Anspruch 1, **dadurch gekennzeichnet, dass** die Untersuchungsprobe eine biologische Proteinprobe darstellt und das nachzuweisende Antigen ein pathogenes Protein-Antigen darstellt.

3. Verfahren zum schnellen Nachweis eines Antigens gemäß Anspruch 2, **dadurch gekennzeichnet, dass** das pathogene Protein-Antigen ein abnormales Prion darstellt.

4. Verfahren zum schnellen Nachweis eines Antigens gemäß Anspruch 1, **dadurch gekennzeichnet, dass** die Untersuchungsprobe aus Lebensmittelmaterial besteht und das nachzuweisende Antigen ein schädliches im Lebensmittelmaterial enthaltenes Protein-Antigen darstellt.

5. Verfahren zum schnellen Nachweis eines Antigens gemäß einem der Ansprüche 1 bis 4, **dadurch gekennzeichnet, dass** der Nachweis eines Antigens mit Hilfe von Fluoreszenz-Korrelations-Spektroskopie oder Fluoreszenz-Kreuzkorrelations-Spektroskopie ohne einen Verfahrensschritt vollzogen wird, in dem das in der Untersuchungsprobe befindliche Antigen physikalisch getrennt wird.

## Revendications

1. Procédé de détection rapide d'un antigène dans un échantillon par spectroscopie à corrélation de fluorescence ou par spectroscopie à corrélation croisée de fluorescence, comprenant un processus consistant à (1) ajouter à un échantillon d'essai un anticorps intact marqué par fluorescence ou un fragment d'anticorps marqué par fluorescence ciblé sur un épitope d'un antigène devant être détecté et un anticorps intact non marqué par fluorescence ou un anticorps intact marqué par fluorescence ou un fragment d'anticorps marqué par fluorescence ciblé sur un autre épitope de l'antigène en tant que réactif de détection, laisser la réaction antigène-anticorps avoir lieu, et détecter un trimère antigène-anticorps formé par spectroscopie à corrélation de fluorescence ou par spectroscopie à corrélation croisée de fluorescence et un processus consistant à (2) prédire par calcul un rapport de concentration entre le réactif de détection et l'antigène auquel une concentration de trimère maximale est atteinte et ajouter à l'échantillon d'essai un antigène et le réactif de détection pour atteindre une concentration de trimère maximale, suivant une quantité prédite par calcul à partir du rapport de concentration entre le réactif de détection et l'antigène auquel une concentration de trimère maximale est atteinte, laisser la réaction antigène-anticorps avoir lieu, et détecter un trimère antigène-anticorps formé par spectroscopie à corrélation de fluorescence ou par spectroscopie à corrélation croisée de fluorescence, lequel procédé comprend en outre le fait de combiner une présence ou une absence d'un signal de détection de trimère dans les deux processus afin de déterminer une présence ou une absence de l'antigène dans l'échantillon d'essai.

2. Procédé de détection rapide d'un antigène selon la revendication 1, dans lequel l'échantillon d'essai est un échantillon de protéine biologique et l'antigène devant être détecté est un antigène de protéine pathogène.

3. Procédé de détection rapide d'un antigène selon la revendication 2, dans lequel l'antigène de protéine pathogène est un prion anormal.

4. Procédé de détection rapide d'un antigène selon la revendication 1, dans lequel l'échantillon d'essai est un produit alimentaire et l'antigène devant être détecté est un antigène de protéine nocif contenu dans le produit alimentaire.

5. Procédé de détection rapide d'un antigène selon l'une quelconque des revendications 1 à 4, dans lequel la détection d'un antigène par spectroscopie à corrélation de fluorescence ou par spectroscopie à corrélation croisée de fluorescence est réalisée sans avoir recours à un processus de séparation physique de l'antigène contenu dans l'échantillon d'essai.
